Europäisches Patentamt

European Patent Office

Office européen des brevets

(19)

(11) Publication number: **0 331 088**
**A2**

## EUROPEAN PATENT APPLICATION

(12)

(21) Application number: 89103432.4

(22) Date of filing: 27.02.89

(51) Int. Cl.⁴: **A61K 37/02 , C07K 15/06 , A61K 35/22**

(30) Priority: 29.02.88 JP 46705/88

(43) Date of publication of application:
06.09.89 Bulletin 89/36

(84) Designated Contracting States:
BE CH DE ES FR GB IT LI NL SE

(71) Applicant: GREEN CROSS CORPORATION
15-1, Imabashi 1-chome Higashi-ku
Osaka-shi
Osaka(JP)

Applicant: MORINAGA MILK INDUSTRY CO.,
LTD.
33-1, Shiba 5-chome
Minato-ku, Tokyo 108(JP)

(72) Inventor: Masaoka, Tohru
17-23, Hata 1-chome
Ikeda-shi Osaka(JP)

(74) Representative: Kolb, Helga, Dr. Dipl.-Chem. et
al
Hoffmann, Eitle & Partner Patentanwälte
Arabellastrasse 4
D-8000 München 81(DE)

(54) Remedy for hematopoietic tissue diseases comprising human monocytic macrophage colony stimulating factor as active ingredient.

(57) A remedy for hematopoietic tissue diseases comprising human monocytic macrophage colony stimulating factor, which promotes the differentiation and multiplication of monocytic macrophages, as an active ingredient is disclosed. This remedy is to be administered before or after bone marrow transplantation for treating those who suffer from hematopoietic tissue diseases.

EP 0 331 088 A2

# REMEDY FOR HEMATOPOIETIC TISSUE DISEASES COMPRISING HUMAN MONOCYTIC MACROPHAGE COLONY STIMULATING FACTOR AS ACTIVE INGREDIENT

## FIELD OF THE INVENTION

This invention relates to a remedy for hematopoietic tissue diseases. More particularly, it relates to a remedy for hematopoietic tissue diseases comprising human monocytic macrophage colony stimulating factor to be administered before or after bone marrow transplantation, which exerts its effects on human monocytic macrophage cells as well as stem cells thereof to thereby promote the differentiation and multiplication of the monocytic macrophages.

## BACKGROUND OF THE INVENTION

A colony stimulating factor, which will be abbreviated as CSF hereinafter, is a hematopoietic factor which occurs in mammal hematopoietic tissues such as bone marrow and promotes the differentiation and multiplication of hematopoietic stem cells. Many CSFs comprise glycoproteins. There have been known four CSFs, namely, M-CSF or CSF-1 which acts on monocytic macrophage stem cells; GM-CSF which acts on granulocytic/monocytic stem cells; G-CSF which acts on granulocytic stem cells; and Multi-CSF, interleukin-3 or IL-3 which acts on multipotential stem cells common to granulocytes, monocytes, erythrocytes and megakaryocytes.

Bone marrow transplantation aims at transplanting normal stem cells, i.e., bone marrow to a patient carrying damaged immunocompetent cells or hematopoietic cells to thereby reconstitute the damaged function. Recently, bone marrow transplantation has been widely applied not only to the treatment of those suffering from primary or secondary damage or defect in hematopoietic tissues or immunocompetent cells but also with immunosuppression therapy for malignant tumors to thereby establish an enhanced antitumor effect by reconstituting the damaged tissue or cells. Therefore, bone marrow transplantation is effective not only on primary immunodeficiency, aplastic anemia and progessive hereditary hemocytic dysfunction but also on hematopoietic tissue tumors such as acute leukemia, chromic leukemia, malignant lymphoma, plasmacytoma and progressive solid tumor. Although bone marrow transplantation is a highly useful method in treating hematopoietic tissue diseases, as described above, the insufficient function of the transplanted bone marrow frequently results in, for example, infectious diseases, interstitial pneumonia or graft versus host disease (GVHD).

Known substances for promoting the bone marrow function after bone marrow transplantation include HGI-glycoprotein (cf. EP-A-212501) and GM-CSF [cf. A.W. Nienhuis et al., J. of Clin. Invest., 80, 573 - 577 (1987)].

## SUMMARY OF THE INVENTION

Bone marrow transplantation for treating a hematopoietic tissue disease is often conducted at a very low leukocyte blood level. This low leukocyte level remains for a considerably long period after the completion of the transplantation, and the patient is in danger of suffering from, for example, infectious diseases throughout this period. Under these circumstances, we have attempted to rapidly elevate the leukocyte level immediately after the transplantation by promoting the production of leukocytes. As a result, we have found that the blood leukocyte level can be rapidly restored to the normal level by administering the human monocytic macrophage colony stimulating factor according to the present invention before or after the bone marrow transplantation, thus completing the present invention.

Accordingly, the present invention provides a remedy for hematopoietic tissue diseases which comprises human monocytic macrophage colony stimulating factor as an active ingredient. The present invention further provides a remedy for hematopoietic tissue diseases comprising human monocytic macrophage colony stimulating factor which acts on mammal monocytic macrophage cells and stem cells thereof to thereby stimulate the differentiation and multiplicaiton of the monocytic macrophages.

The present invention furthermore provides a remedy for hematopoietic tissue diseases wherein said human monocytic macrophage colony stimulating factor, namely, the active ingredient, is obtained from human urine, a culture medium of human monocytic macrophage colony stimulating factor-producing cells or a culture medium of human monocytic macrophage colony stimulating factor-producing recombinant cells. Furthermore, the present invention provides a remedy for hematopoietic tissue diseases comprising mammal monocytic macrophage colony formation stimulating factor having the following physicochemical properties. This factor is described in EP-A-276511 and a process for preparing the same and the physicochemical properties of the same are described in detail in the above publication.

a) Molecular weight

It is a homodimer composed of two identical subunits. Its molecular weight determined by sodium dodecyl sulfate polyacrylamide gel electrophoresis is 70,000 to 90,000 daltons. The molecular weight of a subunit, which does not retain biological activity resulting from dissociation with a reducing agent, determined by sodium dodecyl sulfate polyacrylamide gel electrophoresis is 35,000 to 45,000 daltons.

b) Amino acid sequence of subunit

The subunit protein of the homodimer comprises the following amino acid sequences involving 214 to 238 amino acids (at least amino acids 1 to 214 being present, or functional equivalent thereof). The 122nd and 140th asparagines each show a typical N-glucoside linkage represented by asparagine (Asp)-x-threonine (Thr)/serine (Ser) wherein x represents a permissive amino acid.

```
1
Glu-Glu-Val-Ser-Glu-Tyr-Cys-Ser-His-Met-Ile-Gly-Ser-Gly--His-Leu-Gln-Ser-Leu-Gln-Arg-Leu-Ile-Asp-Ser-Gln-Met-Glu--Thr-Ser-Cys-Gln-Ile-Thr-Phe-Glu-Phe-Val-Asp-Gln-Glu-Gln-
                                   50
-Leu-Lys-Asp-Pro-Val-Cys-Tyr-Leu-Lys-Lys-Ala-Phe-Leu-Leu--Val-Gln-Asp-Ile-Met-Glu-Asp-Thr-Met-Arg-Phe-Arg-Asp-Asn--Thr-Pro-Asn-Ala-Ile-Ala-Ile-Val-Gln-Leu-Gln-Glu-Leu-Ser--Leu-Arg-Leu-Lys-Ser-Cys-Phe-Thr-Lys-Asp-Tyr-Glu-Glu-His-
       100
-Asp-Lys-Ala-Cys-Val-Arg-Thr-Phe-Tyr-Glu-Thr-Pro-Leu-Gln--Leu-Leu-Glu-Lys-Val-Lys-Asn-Val-Phe-Asn-Glu-Thr-Lys-Asn--Leu-Leu-Asp-Lys-Asp-Trp-Asn-Ile-Phe-Ser-Lys-Asn-Cys-Asn-
                                   150
-Asn-Ser-Phe-Ala-Glu-Cys-Ser-Ser-Gln-Asp-Val-Val-Thr-Lys-

-Pro-Asp-Cys-Asn-Cys-Leu-Tyr-Pro-Lys-Ala-Ile-Pro-Ser-Ser-
```

```
-Asp-Pro-Ala-Ser-Val-Ser-Pro-His-Gln-Pro-Leu-Ala-Pro-Ser-

-Met-Ala-Pro-Val-Ala-Gly-Leu-Thr-Trp-Glu-Asp-Ser-Glu-Gly-
                       200
-Thr-Glu-Gly-Ser-Ser-Leu-Leu-Pro-Gly-Glu-Gln-Pro-Leu-His-
                       214
-Thr-Val-Asp-Pro-Gly-Ser-Ala-Lys-Gln-Arg-Pro-Pro-Arg-Ser-
                                                        238
-Thr-Cys-Gln-Ser-Phe-Glu-Pro-Pro-Glu-Thr-Pro-Val-Val-Lys
```

c) Isoelectric point

Its isoelectric point (pI) determined by polyacrylamide gel isoelectric focusing and sucrose density gradient isoelectric focusing is 3.1 to 3.7.

d) Glycoprotein constituting sugar chain

Analysis by high performance liquid chromatography following hydrolysis indicates that the sugar chain comprises mannose, galactose, N-acetylgalactosamine and N-acetylneuraminic acid.

e) Circular dichroism spectrum

Its far ultraviolet CD spectrum determined by using a dichroism dispersion meter shows minimum peaks at the wavelengths 208 nm and 222 nm, suggesting an $\alpha$-helix structure.

f) Thermal stability

Its biological activities are not lost when heated at $60 \pm 0.5\,^{\circ}C$ for 60 minutes.

g) Infrared absorption spectrum

Its infrared absorption spectrum shows intense absorptions at wave numbers 1680 cm$^{-1}$, 1200 cm$^{-1}$ and 1130 cm$^{-1}$ and moderate ones at wave numbers 1540 cm$^{-1}$, 1430 cm$^{-1}$ and 1070 cm$^{-1}$.

The CSF of the present invention having the abovementioned physicochemical properties comprises a biologically active homodimer wherein two polypeptides involving sugar chains are bound to each other through a disulfide bond, similar to CSF-1 [cf. Science, 235, 1504-1508 (1989)]. Its molecular weight ranges from 70,000 to 90,000 daltons, namely, larger than that of CSF-1. The polypeptide of the subunit constituting the CSF of the present invention comprises at least 214 to 238 amino acids and has a molecular weight of 21,400 daltons which is larger than that of CSF-1, i.e., 14,000 to 17,000 daltons. When the amino acid sequence of the subunit of the CSF of the present invention is compared with that of CSF-1, the 1st to 149th, from the NH2-terminal, amino acids of the former show the same sequence at those of the latter but the sequence of the 150th to 214th amino acids of the former is different from that estimated from the cDNA of the latter. Thus, the amino acid sequence of the former is not coded on the CSF-1 gene. These facts obviously indicate that the CSF of the present invention is different from the known CSF-1. In addition, the abovementioned known HGI-glycoprotein and GM-CSF each show completely different biological activities and physicochemical properties from those of the CSF of the present invention.

## BRIEF DESCRIPTION OF THE DRAWINGS

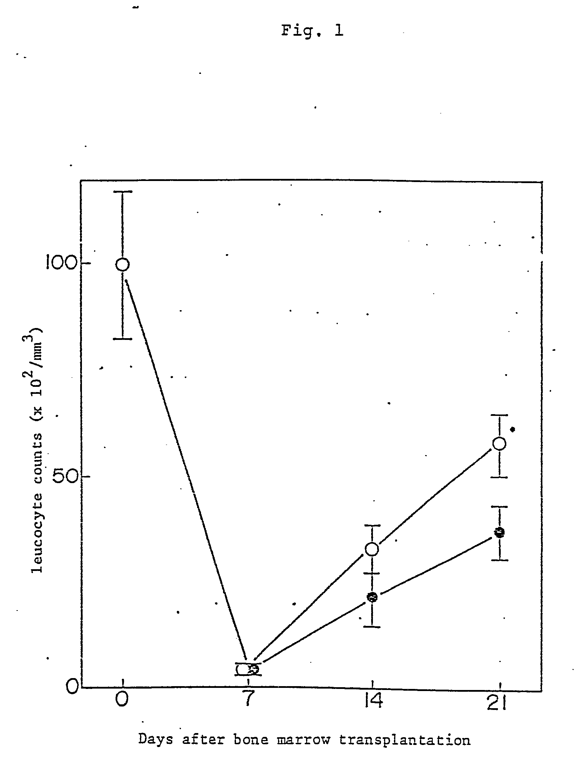

Fig. 1 plots the effect of promoting the restoration of leukocytes when the remedy for hematopoietic tissue disease of the present invention is administered to $^{60}$Co-irradiated mice subjected to bone marrow transplantation as described in Example 3 hereinbelow, by counting the peripheral leukocytes of the animals with the lapse of time: wherein O shows a test group while ● shows a control group to which physiological saline solution is administered. ● shows a significant difference at a significant level of 5 %.

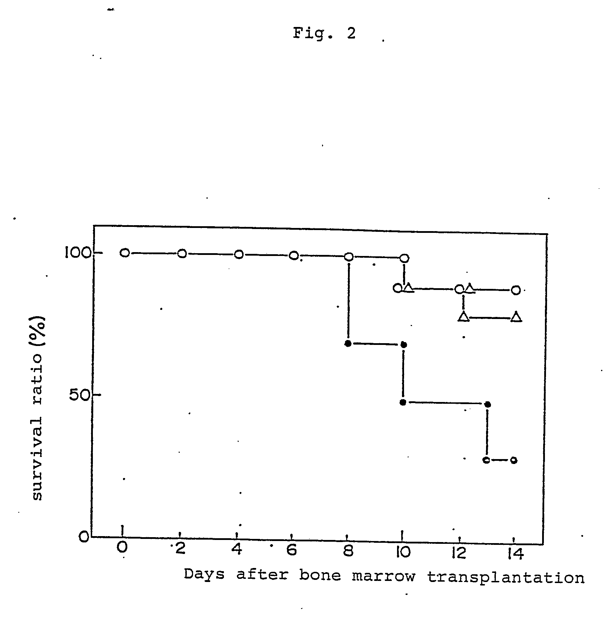

Fig. 2 plots the effect of lowering the mortality of mice with the lapse of time when the remedy for hematopoietic tissue disease of the present invention is administered to $^{60}$Co-irradiated mice subjected to low concentration bone marrow transplantation, before or after the bone marrow transplantation, as described in Example 4 hereinbelow: wherein O shows a pretransplantation administered group; △ shows a post-transplantation administered group; and ● shows a control group.

## DETAILED DESCRIPTION OF THE INVENTION

### (1) Preparation of remedy for hematopoietic tissue diseases

The remedy for hematopoietic tissue diseases of the present invention having the abovementioned properties may be prepared by, for example, the following process. The pH value of the urine of a normal subject is adjusted to 8.0 to 9.0, enabling viscous matters therein to be removed by precipitation. The supernatant is concentrated and desalted by using an ultrafilter capable of passing materials of 10,000 to 50,000 daltons in molecular weight. After concentrating it at least 200-fold (protein concentration: 1 % (w/v) or above), the pH value of the concentrate is adjusted to 6.5 to 7.5. Then, it is heated at 60°C for ten hours to thereby inactivate, for example, viruses contained therein. The precipitate thus formed is removed by centrifuging and the active ingredient is adsorbed by an anion exchanger such as DEAE/cellulose.

Then, the anion exchanger is washed with a 0.05 to 0.1 M buffer solution (pH 6.5 - 7.5) and the active ingredient is eluted with a 0.2 to 0.4 M buffer solution (pH 6.5 - 7.5). The eluate is concentrated with an ultrafilter, if required, and equilibrated with a 1 to 4 M buffer solution (pH 6.5 - 7.5) containing a salt such as ammonium sulfate or sodium chloride. Then, it is subjected to gel filtration with the use of a gel filter such as Sephacryl S-300 (mfd.by Pharmacia). Then, a fraction of 70,000 to 150,000 in molecular weight is collected. This fraction is adsorbed by a hydrophobic affinity material such as Phenyl-Sepharose® (mfd. by Pharmacia) previously equilibrated with the abovementioned 1 to 4 M salt-containing buffer and then eluted with a 0.5 to 1.0 M salt-containing buffer solution (pH 6.5 - 7.5). The eluate is concentrated by ultrafiltration and then subjected to gel filtration by using high performance liquid gel column such as TSKG-3000 SW (mfd. by Tosoh Corporation). A fraction of 70,000 to 150,000 in molecular weight is collected. This fraction is concentrated again and adsorbed by a reverse phase high performance liquid column such as H-Pore® 214TP (mfd. by Vydac) equilibrated with 0.1 % trifluoroacetic acid (TFA) solution (pH 1 - 2). Then, it is subjected to linear gradient elution by using a solvent such as acetonitirle or isopropanol containing 0.1 % TFA. The active fraction is collected and lyophilized. The CSF preparation of the present invention thus obtained is a pure material showing a specific activity of $1 \times 10^8$ U/mg protein or above. The lyophilized fraction is dissolved in a 0.001 to 0.2 M buffer solution (pH 6.5 - 7.5) containing 0.1 to 10 w/v% of human serum albumin and 0.1 to 10 w/v % of sugars (e.g., monosaccharide such as glucose, disaccharide such as sucrose, sugar alcohol such as mannitol, etc.), which is used as a stabilizer for the human monocytic macrophage colony stimulating factor. The obtained solution is aseptically filtered and aseptically lyophilized to thereby provide a remedy for hematopoietic tissue diseases.

The preparations according to the present invention are dissolved in, e.g., physiological saline, and/or distilled water, for injection, etc., at a concentration of from 10 to 100 mg/ml and administered by drip infusion or direct intravenous, intramuscular or subcutaneous injection.

The dosage for human patient usually ranges from 1,000 to 150,000 units/Kg body weight per dose but is subject to variation according to symptoms.

In case of pretransplantation, the time of administration is from immediately before the marrow transplantation or 1 to 7 days before the transplantation to the day of the transplantation. In case of post-transplantation, the time of administration is immediately after the marrow transplantation or 3 to 10 days after the marrow transplantation, at which the leucocyte level does not reach the normal value or a GVH (graft vs host) reaction to the implant seems to occur. In both case, the administration may be maintained for several days (2 to 14 days) according to a variation in a leucocyte level until it becomes constant.

The biological activities of a product obtained in accordance with the following Reference Example 1, which illustrates a process for preparing the remedy for hematopoietic tissue diseases of the present invention, is presented as an example of an embodiment of the present invention.


REFERENCE EXAMPLE 1


The pH value of 1000 l of urine from a normal subject was adjusted to 8.5. The precipitate was filtered off and the filtrate was concentrated and desalted with the use of an ultrafilter (H10X50 mfd. by Amicon; fractionating molecular weight: 50,000 daltons). After adjusting the pH value of the obtained concentrate to 7.0, it was sterilized by heating in a sealed container at 60°C for ten hours. After the completion of the sterilization, it was centrifuged at 5,000 x g for 30 minutes and the precipitate thus formed was removed. The residue was adsorbed by DEAE/cellulose equilibrated with a 0.02 M phosphate buffer solution (pH 7.2) by mixing therewith. The DEAE/cellulose was washed with a 0.02 M phosphate buffer solution and a 0.02 M phosphate buffer solution (pH 7.2) containing 0.05 M of sodium chloride and then eluted with a 0.25 M phosphate buffer solution (pH 7.2) containing sodium chloride. The eluate was concentrated with the use of an ultrafilter (H10P10 mfd. by Amicon) and the concentrate was subjected to gel filtration by using Sephacryl S-300 (mfd. by Pharmacia; 20 cm (i.d.) x 80 cm (h)) and a 1 M buffer solution (pH 7.2) containing ammonium sulfate. A fraction of 70,000 to 150,000 daltons in molecular weight obtained from the gel filtration was applied to a Phenyl-Sepharose 4B column (mfd. by pharmacia; 10 cm (i.d.) x 20 cm (h)) previously equilibrated with the abovementioned 1 M buffer solution containing ammonium sulfate for adsorption and then eluted with a 0.5 M buffer solution (pH 7.2) contaniing ammonium sulfate. The eluate was concentrated with the use of an ultrafilter (H1P10 mfd. by Amicon) and subjected to high performance liquid chromatography by using a TSKG-3000 SW column (mfd. by Tosoh Corporation, 2.5 cm (i.d.) x 60 cm (h)) to thereby give a fraction of 70,000 to 150,000 in molecular weight. This fraction was concentrated again and the desired CSF was eluted by high performance liquid chromatography under linear concentration gradient of 0 to 100 % acetonitrile (pH 2.0) containing 0.1 % of TFA in a reverse column of Hi-Pore 214TP (mfd. by Vydac, 2.2 cm (i.d.) x 25 cm (h)). After lyophilizing, approximately 4 mg of the CSF of the present invention was obtained. This procedure was repeated to thereby give 20 mg of the CSF of the present invention. Then, the lyophilized fraction was dissolved in a 0.02 M phosphate buffer solution (pH 7.2) contaniing 1 w/v% of human serum albumin and 5 w/v% of mannitol, which was used as a stabilizer. The resulting mixture was sterilized by using an aseptic filter (mfd. by Milipore) provided with a membrane filter of 0.22 μm in pore size. Then, it was aseptically pipetted into glass vials, which had been sterilized by heating to 180°C for two hours, by 1 ml portions and aseptically lyophilized. These vials were sealed and thus approximately 500 vials containing the remedy for hematopoietic tissue diseases comprising the CSF of the present invention (4,000,000 units/vial) were obtained.


REFERENCE EXAMPLE 2


The procedure of Reference Example 1 was repeated except that the Sephacryl S-300 was replaced with TSKG 3,000SW (mfd. by Tosoh Corporation) and the Phenyl-Sepharose 4B was replaced with TSK phenyl-5PW (mfd. by Tosoh Corporation). The CSF of the present invention, which was purified by high performance liquid chromatography, was aseptically filtered and lyophilized by the same methods as those described in Reference Example 1. Thus, approximately 500 vials containing the remedy for hematopoietic tissue diseases comprising the CSF of the present invention (4,000,000 units/vial) were obtained.


EXAMPLE 1


Colony stimulating activity and specific activity


The colony stimulating activity of the CSF of the present invention prepared in Reference Example 1 was determined by a colony formation test in vitro by using mouse myeloid cells in a single layer soft agar

6

gel. A sample (0.1 μg of protein) was mixed with 1 ml of McCoy's 5A medium containing 0.3 % of agar, 20 % of fetal calf serum (FCS) and 1 x $10^5$ mouse myeloid cells. Then, the cells were incubated under a 7.5 % $CO_2$ stream at 37°C for seven days. After the completion of the incubation, cell masses involving 50 or more cells were regarded as colonies. The colonies thus formed were counted.

The colony stimulating activity was expressed in units by referring to the amount of the CSF required for the formation of one colony as 1 U. The specific activity was expressed in the number of colonies (U) formed per mg of the CSF. As a result, the CSF of the present invention showed a specific activity of 1.4 x $10^8$ U/mg protein. The obtained colonies were stained with hamatoxylin-eosin and morphologically classified. Consequently, more than 95 % of the colonies comprised monocytic macrophages.

## EXAMPLE 2

Effect of promoting the multiplication of mouse myeloid monocytic macrophage stem cells (CFU-M) in vivo

The product of the present invention in doses of 80 x $10^4$ U/kg body weight, 160 x $10^4$ U/kg body weight, 320 x $10^4$ U/kg body weight and 640 x $10^4$ U/kg body weight and physiological saline solution showing a colony stimulating activity of 0 U/kg body weight were intraperitoneally administered to $C_{57}$BL mouse groups, each having five animals, once a day continuously for three days. On the day after the final administration, the femur and the spleen of each animal were taken out and monocytic macrophage stem cells (CFU-M) in the bone marrow and spleen were counted by the abovementioned soft agar plate test with the use of 1,000 U of the CSF as a stimulating factor. Tables 1 and 2 show the results.

Table 1

| Effect of promoting the multiplication of mouse bone marrow CFU-M | |
|---|---|
| Dose (x $10^4$ U/kg) | No. of CFU-M (x $10^4$/bone marrow) |
| 0 | 3.05 ± 0.65 |
| 80 | 3.82 ± 0.66 |
| 160 | 5.23 ± 0.36* |
| 320 | 5.81 ± 0.57* |
| 640 | 6.02 ± 0.45* |
| Note: | |

* shows a significant difference at a significant level of 1 %.

Table 2

| Effect of promoting the multiplication of mouse spleen CFU-M | |
|---|---|
| Dose (x $10^4$ U/kg) | No. of CFU-M (x $10^4$/bone marrow) |
| 0 | 1.09 ± 0.42 |
| 80 | 1.25 ± 0.49 |
| 160 | 2.20 ± 0.38* |
| 320 | 1.91 ± 0.74* |
| 640 | 1.85 ± 0.55* |
| Note: | |

\* shows a significant difference at a significant level of 1 %.

As shown in Tables 1 and 2, the administration of the remedy in a dose of 80 x $10^4$ U/kg caused an increase in bone marrow CFU-M as well as that in spleen CFU-M. The administration of the same in a dose of 160 x $10^4$ U/kg caused in each a remarkable increase.

### EXAMPLE 3

Effect of promoting the recovery of bone marrow-transplanted mouse

BALB/c mouse groups, each having five animals, were systemically irradiated with $^{60}$Co at 7.8 Gy. Then, 1.0 x $10^6$ of myeloid cells of mice of the same strain were transplanted via the tail vein of each mouse. During five days following the $^{60}$Co irradiation, the remedy of the present invention in a dose of 640 x $10^4$ U/kg and physiological saline solution having no activity were intraperitoneally administered to the mice once a day. The blood of each animal was collected from the tail vein on the 7th, 14th and 21st days after the bone marrow transplantation and peripheral leukocytes therein were counted. On the 14th and 21st days after the bone marrow transplantation, the femur and spleen of each mice were taken out and the monocytic macrophage stem cells (CFU-M) therein were counted by forming colonies according to the abovementioned soft agar plate test by using 1,000 U of the CSF as a stimulating factor. Fig. 1 and Table 3 show the results. The physiological saline solution (0 U/kg) was administered to the control group.

Table 3

| Effect of promoting recovery of CFU-M in bone marrow and spleen of bone marrow-transplanted mouse | | |
|---|---|---|
| Dose (x 10⁴ U/kg) | No. of CFU-M on days after bone marrow transplantation | |
| | 14 | 21 |
| Femur | (x 10⁴/femur) | (x 10⁴/femur) |
| 0 | 0.70 ± 0.07 | 0.71 ± 0.13 |
| 640 | 1.01 ± 0.01 | 1.66 ± 0.54* |
| Spleen | (x 10 /spleen) | ( x 10 /spleen) |
| 0 | 3.53 ± 0.72 | 2.13 ± 0.99 |
| 640 | 4.09 ± 0.92 | 4.02 ± 0.75* |
| Note: | | |

*shows a significant difference at a significant level of 5 %.

As shown in Fig. 1 and Table 3, the remedy of the present invention remarkably promoted the recovery of the leukocyte number and monocytic macrophage stem cells (CFU-M) in the bone marrow and spleen of bone marrow-transplanted mice.

## EXAMPLE 4

Effect of lowering the mortality of bone marrow-transplanted mouse at low concentration

cells of mice of the same strain were transplanted to each animal via the tail vein. During five days before or after the bone marrow transplantation, 640 x 10⁴ U/kg of the remedy of the present invention was administered to each animal once a day. The former mice were referred to as the pretransplantation-administered group while the latter ones were referred to as the posttransplantation-adminstered group. No remedy was administered to the control group. During 14 days after the bone marrow transplantation, the mortality of each group was observed. Fig. 2 shows the results.

As shown in Fig. 2, the remedy of the present invention remarkably lowered the mortality of low concentration bone marrow-transplanted mice, when administered to the animals before or after the transplantation.

While the invention has been described in detail and with reference to specific examples thereof, it will be apparent to one skilled in the art that various changes and modifications can be made therein without departing from the spirit and scope thereof.

## Claims

1. The use of a monocytic-macrophage colony stimulating factor comprising a homodimer in which at least one subunit thereof includes the following amino acid sequence:
-Asp-Val-Val-Thr-Lys-Pro-Asp-Cys-Asn-Cys-Leu-Tyr-Pro-Lys--Ala-Ile-Pro-Ser-Ser-Asp-Pro-Ala-Ser-Val-Ser-Pro-His-Gln--Pro-Leu-Ala-Pro-Ser-Met-Ala-Pro-Val-Ala-Gly-Leu-Thr-Trp--Glu-Asp-Ser-Glu-Gly-Thr-Glu-Gly-Ser-Ser-Leu-Leu-Pro-Gly--Glu-Gln-Pro-Leu-His-Thr-Val-Asp-Pro-
or functional equivalent thereof for the manufacture of a medicament for treatment of hematopoietic tissue diseases in which bone marrow transplantation is carried out.

2. The use of claim 1 in which both of the subunits of the homodimer are the same.

3. The use of claim 1 wherein said CSF includes all or part of the following amino acid sequence adjacent the-Pro-terminus of the amino acid sequence of claim 1:

Gly-Ser-Ala-Lys-Gln-Arg-Pro-Pro-Arg-Ser-Thr-Cys--Gln-Ser-Phe-Glu-Pro-Pro-Glu-Thr-Pro-Val-Val-Lys-

4. The use of claim 1 wherein the amino acid sequence adjacent the-Asp-terminus of the amino acid sequence of claim 1 is as follows:

Glu-Glu-Val-Ser-Glu-Tyr-Cys-Ser-His-Met-Ile-Gly-Ser-Gly--His-Leu-Gln-Ser-Leu-Gln-Arg-Leu-Ile-Asp-Ser-Gln-Met-Glu--Thr-Ser-Cys-Gln-Ile-Thr-Phe-Glu-Phe-Val-Asp-Gln-Glu-Gln--Leu-Lys-Asp-Pro-Val-Cys-Tyr-Leu-Lys-Lys-Ala-Phe-Leu-Leu--Val-Gln-Asp-Ile-Met-Glu-Asp-Thr-Met-Arg-Phe-Arg-Asp-Asn--Thr-Pro-Asn-Ala-Ile-Ala-Ile-Val-Gln-Leu-Gln-Glu-Leu-Ser--Leu-Arg-Leu-Lys-Ser-Cys-Phe-Thr-Lys-Asp-Tyr-Glu-Glu-His--Asp-Lys-Ala-Cys-Val-Arg-Thr-Phe-Tyr-Glu-Thr-Pro-Leu-Gln--Leu-Leu-Glu-Lys-Val-Lys-Asn-Val-Phe-Asn-Glu-Thr-Lys-Asn--Leu-Leu-Asp-Lys-Asp-Trp-Asn-Ile-Phe-Ser-Lys-Asn-Cys-Asn--Asn-Ser-Phe-Ala-Glu-Cys-Ser-Ser-Gln.

5. The use of claim 1 wherein the CSF has the following physicochemical properties:

a) Molecular weight

It is a homodimer composed of two identical subunits. Its molecular weight determined by sodium dodecyl sulfate polyacrylamide gel electrophoresis is 70,000 to 90,000 daltons. The molecular weight of a subunit, which has been biologically inactivated by dissociating with a reducing agent, determined by sodium dodecyl sulfate polyacrylamide gel electrophoresis is 35,000 to 45,000 daltons;

b) Amino acid sequence of subunit

The subunit protein consisting the homodimer has the following amino acid sequences involving 214 to 238 amino acids. The 122nd and 140th asparagines show each a typical N-glucoside linkage represented by asparagine (Asp)-x-threonine (thr)/serine (Ser) wherein x represents a permissive amino acid:

```
1
Glu-Glu-Val-Ser-Glu-Tyr-Cys-Ser-His-Met-Ile-Gly-Ser-Gly--His-Leu-Gln-Ser-Leu-Gln-Arg-Leu-Ile-Asp-Ser-Gln-Met-Glu--Thr-Ser-Cys-Gln-Ile-Thr-Phe-Glu-Phe-Val-Asp-Gln-Glu-Gln-
                              50
-Leu-Lys-Asp-Pro-Val-Cys-Tyr-Leu-Lys-Lys-Ala-Phe-Leu-Leu--Val-Gln-Asp-Ile-Met-Glu-Asp-Thr-Met-Arg-Phe-Arg-Asp-Asn--Thr-Pro-Asn-Ala-Ile-Ala-Ile-Val-Gln-Leu-Gln-Glu-Leu-Ser--Leu-Arg-Leu-Lys-Ser-Cys-Phe-Thr-Lys-Asp-Tyr-Glu-Glu-His-
    100
-Asp-Lys-Ala-Cys-Val-Arg-Thr-Phe-Tyr-Glu-Thr-Pro-Leu-Gln--Leu-Leu-Glu-Lys-Val-Lys-Asn-Val-Phe-Asn-Glu-Thr-Lys-Asn--Leu-Leu-Asp-Lys-Asp-Trp-Asn-Ile-Phe-Ser-Lys-Asn-Cys-Asn-
                                        150
-Asn-Ser-Phe-Ala-Glu-Cys-Ser-Ser-Gln-Asp-Val-Val-Thr-Lys-

-Pro-Asp-Cys-Asn-Cys-Leu-Tyr-Pro-Lys-Ala-Ile-Pro-Ser-Ser-

-Asp-Pro-Ala-Ser-Val-Ser-Pro-His-Gln-Pro-Leu-Ala-Pro-Ser-

-Met-Ala-Pro-Val-Ala-Gly-Leu-Thr-Trp-Glu-Asp-Ser-Glu-Gly-
                200
-Thr-Glu-Gly-Ser-Ser-Leu-Leu-Pro-Gly-Glu-Gln-Pro-Leu-His-
```

```
                        214
-Thr-Val-Asp-Pro-Gly-Ser-Ala-Lys-Gln-Arg-Pro-Pro-Arg-Ser-
                                                      238
-Thr-Cys-Gln-Ser-Phe-Glu-Pro-Pro-Glu-Thr-Pro-Val-Val-Lys
```

c) Isoelectric point

Its isoelectric point (pI) determined by polyacrylamide gel isoelectric focusing and sucrose density gradient isoelectric focusing is 3.1 to 3.7;

d) Glycoprotein constituting sugar chain

Analysis by high performance liquid chromatography following hydrolysis indicates that the sugar chain comprises mannose, galactose, N-acetylgalactosamine and N-acetylneuraminic acid;

e) Circular dichroism spectrum

Its far ultraviolet CD spectrum determined by using a dichrograph shows minimum peaks at wavelengths 208 nm and 222 nm, suggesting an $\alpha$-helix structure;

f) Thermal stability

It does not lose the biological activities when heated at 60 ± 0.5 C for 60 minutes; and

g) Infrared absorption spectrum

Its infrared absorption spectrum shows intense absorptions at wave numbers 1680 cm$^{-1}$, 1200 cm$^{-1}$ and 1130 cm$^{-1}$ and moderate ones at wave numbers 1540 cm$^{-1}$, 1430 cm$^{-1}$ and 1070 cm$^{-1}$.

Fig. 1

Days after bone marrow transplantation

Fig. 2

Days after bone marrow transplantation